# EUROPEAN PATENT APPLICATION

(11) **EP 4 778 506 A1**
(43) Date of publication of application: **22.07.2026**
(21) Application number: 25152759.4
(22) Date of filing: 20.01.2025
(51) Int. Cl.: A61K 8/04, A61K 6/20, A61K 8/19, A61K 8/24, A61K 8/44, A61K 8/86, A61Q 11/00

(54) **CATIONIC ACP HYDROGEL FOR TREATMENT OF TOOTH HYPERSENSITIVITY**

(71) Applicant: Koninklijke Philips N.V., 5656 AE Eindhoven (NL)
(72) Inventor: MAO, Jingliang, Eindhoven (NL)
(74) Representative: Philips Intellectual Property & Standards

(57) **Abstract**

A composition for treating dentin hypersensitivity, comprising: a first component comprising: (i) a first rheology modifying polymer; (ii) a calcium ion source; (iii) a positively charged basic amino acid; and (iv) an acidifying agent, wherein the first component has a pH in the range of about 2.0 to about 7.0; a second component comprising: (i) a second rheology modifying polymer; (ii) a phosphate ion source; and (iii) an alkalizing agent, wherein the second component has a pH in the range of about 7.0 to about 14.0; wherein the first component and the second component are combined into a mixture such that a cationic amorphous calcium phosphate gel composition is created instantly when the pH of the mixture reaches a pH range of about 7.0 to about 9.5.

## Description

### FIELD OF THE DISCLOSURE

The present disclosure is directed generally to a hydrogel composition for treating dentin hypersensitivity including a cationic amorphous calcium phosphate (ACP) gel, methods for using the gel composition, and systems for preparing the gel composition.

### BACKGROUND

Tooth and gum sensitivity are areas of particular concern in oral health care, usually the most prominent concern after tooth cavities. As more people use teeth whitening products, this concern is growing.

Tooth sensitivity, also called dentin hypersensitivity, is characterized as a sharp pain arising from exposed dentin, typically in response to an external stimulus such as clinical, thermal, tactile, evaporative, or osmotic stimuli. It is normally experienced when the root of the tooth has been exposed to the oral environment because of gingival recession.

The most widely accepted explanation of dentin hypersensitivity is the hydrodynamic theory, which suggests that nerve stimulation due to movement of dentinal fluid is the cause of hypersensitivity. When dentin tubules are exposed to the oral environment, fluid movement through dentin tubules, can be sensed by internal tooth nerves. The activation of tooth nerve fibers can cause the feeling of sharp pain. The relationship between dentin hypersensitivity and the patency of dentin tubules has been established in vivo and it has been found that occlusion of the tubules decreases that sensitivity.

The technology and materials previously developed to treat dentin hypersensitivity can be classified into two treatment categories: Treatment I and Treatment II as described below.

Treatment I works by desensitizing the tooth nerves via potassium salts such as potassium nitrate, potassium citrate, potassium chloride, which depolarize the excited nerve fibers, thus numbing the pain. This treatment typically provides short-term relief of dentin hypersensitivity.

Treatment II is based on occlusion of the dentin tubules by plugging dentinal tubules or coating the sensitive dentin area with a thin protective layer of material. Occlusion of open tubules blocks the hydrodynamic mechanism. Treatment II may provide a relatively long-term solution.

Most available dentin hypersensitivity treatment products and methods work by blocking the dentinal tubules and prevent dentinal fluid flow. Examples of such materials and methods include the application of specific dentifrices, dentin adhesives, some antibacterial agents, aldehydes, resin suspensions, fluoride rinses, fluoride varnishes, amorphous calcium phosphate (ACP), strontium fluorides, oxalates, Nd-YAG laser application, bioactive glass, casein phosphopeptides, and Portland cement.

However, the existing products and methods to treat dentin hypersensitivity have one or more problems or disadvantages. For example, existing products typically provide insufficient blockage of open tubules, and generally provide slow diffusion and/or dispersion of the active occlusion ingredients into the open dentin tubules. Additionally, existing products offer weak or low affinity to dentin and provide unstable remineralization and lower efficiency of occlusion of dentin tubules. Further, existing products provide hypersensitivity relief only for a limited time. Many existing dentin hypersensitivity treatments also require expensive dental clinic visits.

Accordingly, existing products and methods are inefficient, short-lived, inconvenient, costly, and/or uncomfortable treatments.

### SUMMARY OF THE DISCLOSURE

There is thus a continued need for improved compositions, methods, and systems to treat dentin hypersensitivity.

Various embodiments and implementations are directed to a composition for treating dentin hypersensitivity including a cationic amorphous calcium phosphate (ACP) gel, methods for using the composition, and systems for preparing the composition. The composition includes a first component comprising a thickening polymer(s), a calcium ion source, a positively charged basic amino acid and an acidifying agent. This first component has a pH in the range of about 2.0 to about 7.0 and is already in a pre-formed gel form. The composition further includes a second component comprising a thickening polymer(s), a phosphate ion source, and an alkalizing agent. This second component has a pH in the range of about 7.0 to about 14.0 and is already in a pre-formed gel form. According to an embodiment, the first component and the second component are mixed/combined into a mixture such that a cationic amorphous calcium phosphate gel composition is created when the pH of the gel mixture is controlled between about 7.0 to about 9.5.

According to an aspect, a composition for treating dentin hypersensitivity is provided. The composition comprises a first hydrogel component comprising: (i) a first rheology modifying polymer, which is dosed enough to make a viscous hydrogel for the first component; (ii) a calcium ion source; (iii) a positively charged basic amino acid; and (iv) an acidifying agent, wherein the first hydrogel component has a pH in the range of about 2.0 to about 7.0; a second hydrogel component comprising: (i) a second rheology modifying polymer , which is dosed enough to make a viscous hydrogel for the second component; (ii) a phosphate ion source; and (iii) an alkalizing agent, wherein the second hydrogel component has a pH in the range of about 7.0 to about 14.0; wherein the first hydrogel component and the second hydrogel component are mixed/combined into a mixture such that a cationic amorphous calcium phosphate gel composition is created instantly when the pH of the mixture is controlled at a pH range of about 7.0 to about 9.5.

According to an embodiment, the first hydrogel component and the second hydrogel component are mixed/combined into a mixture via a mixing tip or a mixing chamber.

According to an embodiment, the first and second rheology modifying polymer is one or more of a poloxamer, carbomer, hydroxyethylcellulose, xanthan gum, and polyvinylpyrrolidone.

According to an embodiment, the calcium ion source is a calcium salt of nitric acid, a calcium salt of aspartic acid, a calcium salt of glutamic acid, or a water-soluble calcium salt of another suitable acid.

According to an embodiment, the positively charged basic amino acid is one or more of lysine, arginine, and histidine.

According to an embodiment, the acidifying agent is phosphoric acid, or other acid compatible with ingredients of the first hydrogel component, such that the first hydrogel component has a pH in the range of about 2.0 to about 7.0.

According to an embodiment, the phosphate ion source is disodium phosphate, monosodium phosphate, or other suitable alkaline water-soluble phosphate salt.

According to an embodiment, the alkalizing agent is sodium hydroxide, potassium hydroxide, or other suitable alkaline compound, such that the second hydrogel component has a pH in the range of about 7.0 to about 14.0.

According to an embodiment, the first hydrogel component and/or the second hydrogel component comprises one or more of a flavoring agent, a sweetener, a solvent, a surfactant, fluoride, and a nerve desensitizing agent. According to an embodiment, the nerve desensitizing agent is a potassium salt.

According to another aspect is an oral health care method for treating dentin hypersensitivity. The method includes: providing a first hydrogel component comprising: (i) a first rheology modifying polymer; (ii) a calcium ion source; (iii) a positively charged basic amino acid; and (iv) an acidifying agent, wherein the first hydrogel component has a pH in the range of about 2.0 to about 7.0; providing a second hydrogel component comprising: (i) a second rheology modifying polymer; (ii) a phosphate ion source; and (iii) an alkalizing agent, wherein the second hydrogel component has a pH in the range of about 7.0 to about 14.0, wherein the first hydrogel component and the second hydrogel component are separately housed hydrogels prior to mixing; combining the first hydrogel component and the second hydrogel component together by simultaneously dispensing the first hydrogel component and the second hydrogel component into a mixing tip or chamber thereby instantly forming a cationic amorphous calcium phosphate (ACP) gel composition with the pH of the mixture at a pH range of about 7.0 to about 9.5; and applying the instantly formed cationic amorphous calcium phosphate gel composition onto the dentin surface of the user's teeth.

According to another aspect is a dosing device for preparing a composition for treatment of dentin hypersensitivity. The device includes: a first compartment containing a first hydrogel component comprising: (i) a first rheology modifying polymer; (ii) a calcium ion source; (iii) a positively charged basic amino acid; and (iv) an acidifying agent, wherein the first hydrogel component has a pH in the range of about 2.0 to about 7.0, and a second compartment containing a second hydrogel component comprising: (i) a second rheology modifying polymer; (ii) a phosphate ion source; and (iii) an alkalizing agent, wherein the second hydrogel component has a pH in the range of about 7.0 to about 14.0; and a dispensing portion configured to receive the first hydrogel component and the second hydrogel component into a mixing tip or chamber and to combine the first hydrogel component and the second hydrogel component into a mixture creating a cationic amorphous calcium phosphate gel composition instantly when the pH of the mixture reaches a pH range of about 7.0 to about 9.5, and wherein the dispensing device has an opening to apply the cationic amorphous calcium phosphate gel composition onto a dentin surface of a user's teeth.

According to an embodiment, the dosing device could include multiple compartments, each compartment comprising one or more compatible ingredients of the first hydrogel component and/or one or more compatible ingredients of the second hydrogel component, and wherein the content of the multiple compartments is dispensed into the mixing tip or chamber to form the cationic amorphous calcium phosphate gel composition.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
FIG. 1 is a flowchart of a method for generating a cationic amorphous calcium phosphate (ACP) gel composition, in accordance with an embodiment.
FIG. 2 is a schematic representation of a composition mixing and/or dispensing device or system, in accordance with an embodiment.

### DETAILED DESCRIPTION OF EMBODIMENTS

The present disclosure describes various embodiments of a composition for treating dentin hypersensitivity including a cationic amorphous calcium phosphate (ACP) gel, methods for using the composition, and systems for preparing the composition. More generally, Applicant has recognized and appreciated that it would be beneficial to provide an improved composition and method for long-term treatment of dentin hypersensitivity. The composition includes a first component in hydrogel form comprising a thickening polymer, a calcium ion source, a positively charged basic amino acid and an acidifying agent. This first component has a pH in the range of about 2.0 to about 7.0. The composition further includes a second component in hydrogel form comprising thickening polymer, a phosphate ion source, and an alkalizing agent. This second component has a pH in the range of about 7.0 to about 14.0. According to an embodiment, the first component and the second component are combined into a mixture such that a cationic amorphous calcium phosphate gel composition is created when the pH of the mixture reaches a pH range of about 7.0 to about 9.5.

Thus, a composition for treating dentin hypersensitivity is disclosed, as well as methods for making such compositions, and systems for preparing such compositions. The disclosed compositions include an instantly formed cationic ACP gel that has several advantages over current market available ACP gels that are not cationic and/or are not instantly formed.

The embodiments and implementations disclosed or otherwise envisioned herein result in an improved pre-formed, cationic ACP hydrogel with stronger dentin affinity and better occlusion aggregates to treat dentine hypersensitivity more effectively. This type of hydrogel can be utilized as either a tooth sensitivity relief treatment (such as a toothpaste), including with common abrasive ingredient(s) or as tooth sensitivity relief gel product for the oral health care industry, among other implementations. Many other uses are possible.

It is known that dentine or tooth surface is negatively charged in nature. Chemically, dentin is composed of approximately 20% of organic material (collagen), 75% of inorganic material (calcium phosphate) and 5% water. The collagen functions like a binder in dentine structure. In a healthy tooth, saliva is naturally effective in reducing dentin hypersensitivity by supplying and carrying calcium and phosphate ions into open dentin tubules to gradually occlude them and form a surface protective layer consisting of precipitate of salivary glycoproteins with calcium phosphate. For people with dentin hypersensitivity, the affected tooth dentine exposes numerous microtubules of around 5 µm in diameter. The microtubules allow some small molecular size fluid or particles migration inside dentine. It is highly beneficial to have composition of dentin occluding material close or similar to dentin's natural composition, i.e., being made of calcium phosphates, proteins (or amino acids, peptides), and water. Thus, a cationic charged ACP hydrogel is desired in terms of binding affinity and occlusion efficiency of dentine tubules.

Amino acids, in particular the three basic amino acids arginine (Arg), lysine (Lys), and histidine (His), are common to all life forms. Arginine is conditionally essential in the human diet due to the factor that its synthesis can be limited under special pathophysiological conditions. Lysine (Lys) and histidine (His) are two of the nine essential amino acids humans cannot synthesize. In chemistry, Arginine (Arg), lysine (Lys), and histidine (His) are water soluble, positively charged (i.e., cationic) in neutral or near neutral pH environment.

Calcium salts such as calcium nitrate salt are soluble in acidic environment but not stable in alkaline condition, and phosphate salts such as disodium phosphate are very soluble in alkaline condition but less in neutral pH. When these two salt parts are mixed under the proper ratios/conditions, a neutral or slightly alkaline pH condition of the final mixture/finished product is created and amorphous calcium phosphate (ACP) formation starts and accelerates with increased pH, since ACP has very poor solubility in neutral or slightly alkaline pH condition. As described herein, the novel ACP is formed by a combination of a two-part system into a single finished product: one part will be water soluble calcium salts of nitric acid, aspartic acid (Asp), glutamic acid (Glu) or other suitable acid, plus other functional or inert ingredient(s), another part will be water soluble phosphate salts like disodium phosphate and/or monosodium phosphate, functional or inert ingredient(s). When basic amino acids like arginine (Arg), lysine (Lys), and histidine (His) are included as a component in the two-part formulation system, a final neutral pH or slightly alkaline pH condition fits well to produce a cationic ACP mixture having high affinity to teeth dentine surface.

In some prior art compositions formed by mixing of two pre-formed gel components before application, one pre-formed gel component contains calcium ions and other functional or inertial ingredient(s), another gel component contains phosphate ions and other functional or inertial ingredient(s). However, these compositions are not cationic and have limited efficacy of hypersensitivity relief. Though prior art CPP-ACP toothpastes can contain cationic CPP and ACP, CPP causes life threatening allergy reactions for some users.

As disclosed herein, a composition for treating dentin hypersensitivity includes a cationic relief ACP hydrogel before application for sensitivity relief treatment. When an acidic hydrogel with rheology modifying polymer(s) (i.e., thickener), calcium ions, arginine and/or other basic acids, other functional or inert ingredient(s) is mixed with an alkaline hydrogel of rheology modifying polymer(s), phosphate ions, other functional or inert ingredient(s), it produces a final hydrogel with cationic amino acids and a large quantities of calcium and phosphate ions and some instantly formed ACP nano scale particles. It generates strong occlusion of dentin tubules by the strong binding complex among dentine, positive charged amino acids, calcium and phosphate ions, and/or formed ACP particles. This deposits a dentin-like mineral as a strong plug within the dentin tubules and as a protective layer on the dentin surface. Since calcium phosphate is chemically more resistant to acid challenge than calcium carbonate in occlusion of dentin tubules, the novel composition is superior in duration of dentin hypersensitivity relief compared to prior art methods. In addition, the neutral or slightly alkaline pH of the cationic relief ACP hydrogel is a safer dental application to help remineralize enamel and exposed dentine.

As disclosed herein, the composition can be made up of two parts, three parts, four parts, or multiple parts. It can be cost-effectively built into two-part system such as the one shown in Table 1 below, although other combinations are possible.

**TABLE 1. Composition of two-part system for a cationic relief ACP hydrogel for treatment of dentin hypersensitivity.**

| Composition of Two-Part System | |
|---|---|
| Part 1 | An acidic hydrogel of rheology modifying polymer(s) (i.e. thickener), calcium salts, arginine and/or other basic amino acids, other functional or inert ingredient(s) |
| Part 2 | An alkaline hydrogel of rheology modifying polymer(s) (i.e. thickener), phosphate salts, other functional or inert ingredient(s) |

According to an embodiment, each part of the composition can be prepared in a pre-formed hydrogel form. Upon mixing of the two parts, the dosing system/device with a mixing tip/chamber can dispense them as a homogenous cationic hydrogel with a final pH within 7.0-9.5 range onto the user's teeth or exposed dentine surface.

According to an embodiment, for a composition made of more than two gel parts, each part may contain one or more ingredients used in the two-part composition system, as long as there is no compatibility issue within each part. The production and dosing system for multi-part (≥ 3 parts) composition of the cationic ACP hydrogel is also possible. This system will accurately control dosage of each part/ingredient. Each part or ingredient in this approach can be supplied in cartridge form to make it easier for dosing control, replacement, change, or product choice during application.

According to an embodiment, the composition described herein takes advantage of the interaction principle of charged particles in physics, specifically that opposites attract and like repels. It also takes advantage of chemical properties of calcium salts of nitrate, ASP, or Glu, or cationic charges of Lys, Arg, and His when all of them are readily soluble in water at neutral pH.

As used herein, the term instant" and "instantly" refer to the short period of time beginning with the mixing the components to the time of formation of the finished gel, which is generally less than about 5 seconds.

As used herein, the term "gel" refers to a homogenous solid aqueous gel-like material, such as a hydrogel having water-swellable polymeric matrices that can absorb a substantial amount of water.

The term "acidifying agent" refers to one or more compounds that when dissolved or dispersed in neutral water having a pH of 7 causes the water to have a pH lower than 7.

The term "alkalizing agent" refers to one or more compounds that when dissolved or dispersed in neutral water having a pH of 7 causes the water to have a pH higher than 7.

As used herein, the term "pH manipulation" refers to a formulation strategy accomplished by a pH change of the mixture when acidic or alkaline component(s) are used in a multi-component composition. The pH manipulation is conducted by raising the pH of the final mixture to an alkaline pH, in particular a pH of about 7.0 to about 9.5, when a predetermined amount of one acidic component hydrogel is mixed with an amount of another alkaline component hydrogel via a mixing tip or in a mixing chamber. During the mixing, the calcium ions and basic amino acids from the acidic component and the phosphate ions from the alkaline component react to produce cationic ACP compounds.

The term "thickening" refers to an increase in the viscosity of the composition by added chemical(s) which is a polymer or polymers in most cases. A thickener is capable of increasing the viscosity of a composition when the composition pH is in the range of 2.0 to 7.0 or 7.0 to 14.0. The thickener is compatible with one or more of the basic amino acids/peptides, phosphate ions, calcium ions, and/or acidic amino acids.

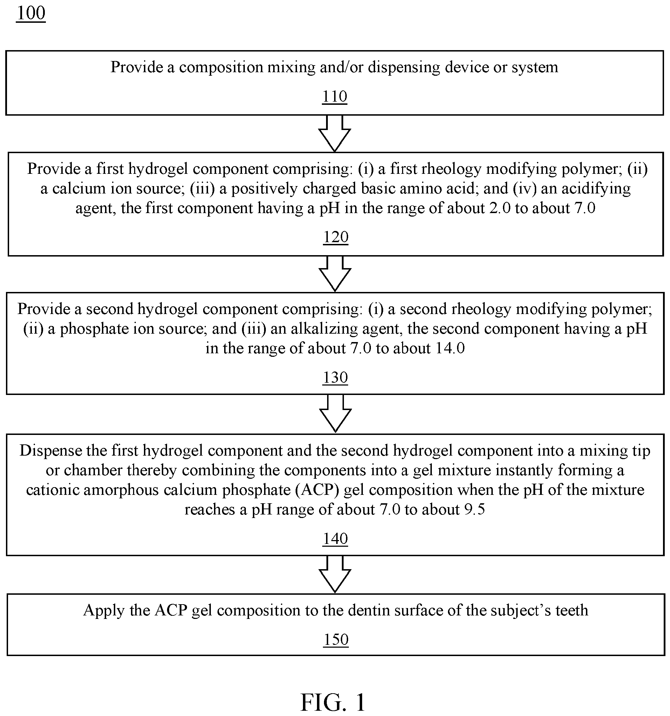

Referring to FIG. 1, in one embodiment, is a flowchart of a method 100 for generating a cationic amorphous calcium phosphate (ACP) gel composition from the combination of two separate composition, and treating a dentin hypersensitivity with the gel composition. The methods described in connection with the figures are provided as examples only, and shall be understood not to limit the scope of the disclosure.

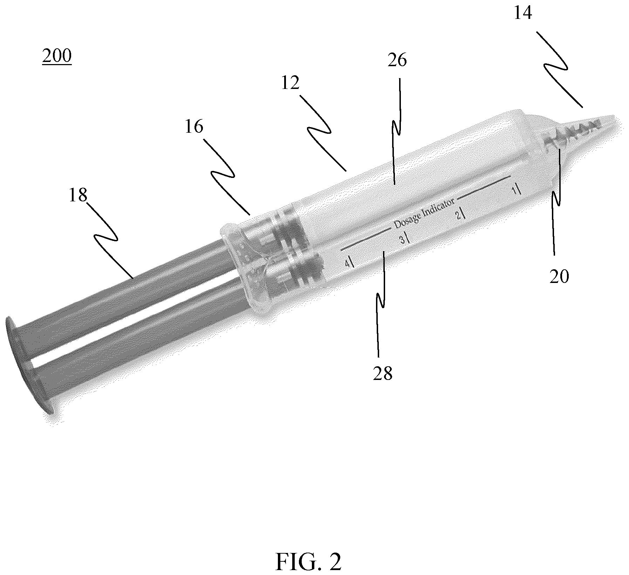

At step 110 of the method, a composition mixing and/or dispensing device or system 200 is provided. Referring to an embodiment of a composition mixing and/or dispensing device or system 200 as depicted in FIG. 2, for example, the device or system comprises a dosing portion 12 and a dispensing portion 14. Dosing portion 12 comprises two or more reservoirs (here shown with two reservoirs), each one of the reservoirs holding a gel component of the composition at the desired mixing ratios. Dosing portion 12 comprises a plunger 18 and a dual-barrel syringe 16 (in this example, although more barrels are possible) wherein one barrel 26 contains a first portion of the mixture before mixing, and the other barrel 28 contains a second portion of the mixture before mixing. Dispensing portion 14 comprises a mixing tip 20 wherein the first portion of the mixture from barrel 26 and the second portion of the mixture from barrel 28 are mixed together to form an instant cationic ACP gel composition when the pH range of combined gel is 7.0 to 9.5.

According to an embodiment, composition mixing and/or dispensing device or system 200 allows the alkaline and the acidic gel components to be stored separately before use. It allows for ingredient stability and avoids premature reaction or binding among ingredients of the two (or more) separate components before they reach the targeted area. When needed, the alkaline and the acidic gel components can be mixed to create a cationic ACP gel at a joint mixing point or chamber before application.

It will be understood that FIG. 2 constitutes, in some respects, an abstraction and that the actual organization of the components of the device or system 200 may be different and more complex than illustrated. Additionally, device or system 200 can be any of the devices or systems described or otherwise envisioned herein. Other elements and components of the device or system 200 are disclosed and/or envisioned elsewhere herein.

Returning to method 100 in FIG. 1, at step 120 of the method a first component is provided, comprising: (i) a first rheology modifying polymer; (ii) a calcium ion source; (iii) a positively charged basic amino acid; and (iv) an acidifying agent, the first component having a pH in the range of about 2.0 to about 7.0. According to an embodiment, the first component is provided via a first barrel or other separate portion of composition mixing and/or dispensing device or system 200.

At step 130 of the method, a second component is provided, comprising: (i) a second rheology modifying polymer; (ii) a phosphate ion source; and (iii) an alkalizing agent, the second component having a pH in the range of about 7.0 to about 14.0. According to an embodiment, the second component is provided via a second barrel or other separate portion of composition mixing and/or dispensing device or system 200.

At step 140 of the method, when ready for application, the first component and the second component are dispensed into a mixing tip or chamber thereby combining the components into a mixture instantly forming a cationic amorphous calcium phosphate (ACP) gel composition when the pH of the mixture reaches a pH range of about 7.0 to about 9.5.

At step 150 of the method, upon or after mixing, the cationic ACP gel composition is applied to the dentin surface of the subject's teeth. The cationic ACP gel composition can be applied using any method for applying a gel to a tooth.

According to an embodiment, the compositions described or otherwise envisioned herein can be made of two parts in composition, although additional parts are possible. The two parts envisioned are as follows:
Part 1: An acidic hydrogel comprising a thickening polymer(s), calcium salts of nitric acid, aspartic acid (Asp), glutamic acid (Glu) or other suitable acid, basic amino acids like Arg (preferred), Lys, or His, acidifying agents, and/or other functional or inert ingredient(s), with a pH of about 2.0 to about 7.0, and preferably about 4.0 to about 6.5. The portion could also comprise one or more other functional agents such as a nerve desensitizing agent like potassium nitrate, flavoring agent like peppermint oil, and more.

Part 2: An alkaline hydrogel of a thickening polymer(s), water soluble phosphate salts, alkalizing agents, plus other functional or inert ingredient(s), with a pH of about 7.0 to about 14.0, and preferably about 8.0 to about 11.5. The portion could also comprise one or more other functional agents such as a nerve desensitizing agent like potassium nitrate, flavoring agent like peppermint oil, and more.

For a two-part composition, the dosing system can be a dual-barrel syringe, dual-chamber bottle, or a dispensing device that can dispense two separate hydrogels to a common mixing tip/chamber to produce final homogenous cationic ACP hydrogel. Such a dual-barrel syringe, dual-barrel bottle, or dispensing device can be formed with two compartments (which can be detached containers/cartridges or integrated chambers in the device) that provide dual reservoirs for the two parts of hydrogel at the mixing ratio as desired. The apparatus further can comprise pump or vacuum means to simultaneously withdraw hydrogel from each compartment, via separate draw pipes, and discharge the hydrogel to a common mixing tip or chamber and after that, to the application areas. This device enables the alkaline and the acidic hydrogel parts of materials to be stored separately before use. It allows for ingredients stability and avoids pre-mature reaction/binding among ingredients of these two separated parts before they reach the targeted area. When needed, the alkaline and the acidic hydrogel parts of materials are mixed & dispensed from a single unit at a joint mixing point or chamber before starting of applications. The desired pH of final homogenous cationic ACP hydrogel is within pH 7.0-9.5.

For dentin hypersensitivity relief, the final cationic ACP hydrogel is applied directly to exposed dentin, optionally massaging for 1-2 minutes to help it diffuse and/or disperse into dentine microtubules. According to one possible embodiment, a cationic ACP hydrogel is applied once or twice a day. The final cationic ACP hydrogel can also be used like a medical toothpaste to be brushed onto teeth to remineralize enamel and treat exposed dentin.

### Thickening Polymers

According to an embodiment, the first component and the second component comprise one or more thickening polymers. There are many non-hazardous, safe rheology modifying polymers suitable such as poloxamer(s), carbomer(s), hydroxyethylcellulose (HEC), xanthan gum, polyvinylpyrrolidone (PVP), an/ or other polymer(s) can be used in one or both parts.

One example is Carbopol^{®} 940 NF polymer. It produces 40,000 - 60,000 cP of viscosity at 0.5% dispersions under pH 7.5- 10.0. Another example is that at pH 2.0-11.0 poloxamer Pluronic F-127 can easily generate 200,000-900,000 cps of viscosity at 28% dosage level in the hydrogel. A preferred dosage of those thickening polymers is between 3%-30% (wt./wt.%), depending on type and molecular weight of thickener chosen.

### Calcium Salts

According to an embodiment, the first component comprises a calcium ion source. The calcium ion source may comprise, for example, calcium salts of nitric acid, aspartic acid (Asp), glutamic acid (Glu), or other suitable acid such as calcium nitrate, aspartic acid calcium, or calcium glutamate, as long as it remains soluble at a pH 2.0-7.0 water solution. The preferred dosage range of calcium salts is 0.5%- 2.0% (wt./wt.%).

### Positively charged basic amino acids

According to an embodiment, the first component comprises one or more positively charged basic amino acids. The three basic amino acids, Lysine, Arginine, and Histidine, have basic side chains at neutral pH. Table 2 shows their pKa1 (α-carboxyl group), pKa2 (α-ammonium ion), pKa3 (side chain R group) and PI (isoelectronic point). The basic amino acids are positively charged at neutral pH condition. Especially arginine and lysine which have 100% positively charged basic side chains between pH 6.5-8.0. Thus, depending on which basic amino acid is used in the composition, it is practical to have strong positive charged basic amino acids around neutral pH. According to an embodiment, among two optical isomers, D(-) and L(+) of lysine, arginine, or histidine, the L form of lysine, arginine, or histidine is preferred. The preferred dosage range of each basic amino acid is 6-18% (wt./wt.%).

**TABLE 2. The pKa1, pKa2, pKa3 and PI of lysine, arginine, and histidine.**

| Basic Amino Acid | pKa1 (α-carboxyl group) | pKa2 (α-ammonium ion) | pKa3 (side chain R group) | pI (isoelectronic point) |
|---|---|---|---|---|
| Lysine | 2.18 | 8.95 | 10.53 | 9.74 |
| Arginine | 2.17 | 9.04 | 12.48 | 10.76 |
| Histidine | 1.82 | 9.17 | 6.00 | 7.59 |

Notably, the basic amino acids can also be formulated as a functional agent in Part 2 when the pH of Part 2 hydrogel isn't highly alkaline, and thus the basic amino acid will remain compatible in Part 2. In formulation, the pH of Part 2 hydrogel needs to be kept away from their PI value when the relevant basic amino acid is used in Part 2.

### Disodium phosphate or other soluble phosphate salts

According to an embodiment, the second component comprises a phosphate ion source. Disodium phosphate, monosodium phosphate, or another alkaline water soluble, non-toxic phosphate salt can be used in the formulation of the second component of the composition. The preferred dosage range is 0.5%-2.0% (wt./wt.%).

### pH Adjusting Agents

Each of the first component and the second component comprise a pH-adjusting agent, with the first component comprising an acidifying agent such that the first component has a pH in the range of about 2.0 to about 7.0, and the second component comprising an alkalizing agent such that the second component has a pH in the range of about 7.0 to about 14.0. According to an embodiment, acidifying agents such as phosphoric acid or other compatible acid could be used and alkalizing agents such as sodium hydroxide, potassium hydroxide can be used, as long as it is compatible with all ingredients in the part it is being used. Please note, if necessary, acidifying agent can also be used to adjust the pH of the second component and alkalizing agent can be used to adjust the pH of the first component.

### Flavoring Agent(s)

According to an embodiment, one or both of the first component and the second component comprise a flavoring agent. Flavoring agents are substances that trigger the sense of taste and/or smell. They are used to improve the consumer perception and experience in treatment of dentine hypersensitivity. Generally, minty flavors such as peppermint oil and spearmint oil are used to give a sense of cleanliness and freshness for dental products. Sweeteners like xylitol or artificial sweeteners like sodium saccharin and sucralose are also used. A commonly preferred flavoring agents is peppermint oil. As long as a flavoring ingredient is compatible with other ingredients in the first component and/or the second component, it could be used at minimum dosage level if it is desired by applications and users.

### Potassium Salts

According to an embodiment, one or both of the first component and the second component comprising a potassium salt. Most quick temporary dentine hypersensitivity desensitizers are potassium based salts, which depolarize the excited nerve fibers and numb the pain. Potassium salts like potassium nitrate, potassium citrate, and potassium chloride could be the preferred choice and thus could be used. In one or both of the first component and the second component hydrogel, potassium salts like potassium nitrate, potassium citrate, and potassium chloride could be used. The preferred dosage range could be ≤ 5.0%.

### Other functional or inert chemical(s) in Part 1 & 2

Depending on what other function(s) is desired for the product, some other functional agents like solvent(s) (e.g., propylene glycol), non-ionic & amphoteric surfactant(s), fluoride, etc., can also be formulated in one or both of the first component and the second component hydrogel to provide some desired function(s) for the composition and desensitizing application. Many such functional agents are possible.

### Dosing system

According to an embodiment, the dosing system/device could be a dual-barrel syringe, dual- chamber bottle, or a dispensing device with two compartments that can dispense hydrogel as a viscous (> 10,000 cps.) gel product via a mixing tip or chamber onto the user's teeth or exposed dentin surface. The apparatus further can comprise pump or vacuum means, with or without sensor/meter, to withdraw contents from the container of each part, via draw pipe, and discharge the final treatment hydrogel contents after homogenous mixing to the targeted tooth or exposed dentine surface area.

### Example Formulation

Table 3 shows one composition formula example of this invention. Other formulas can be readily developed (but not listed here) following the disclosed formulation approach. Thus, this formulation is understood to be a non-limiting embodiment of the compositions described or otherwise envisioned herein.

**TABLE 3. One example formulation of the cationic ACP hydrogel for treatment of hypersensitive teeth.**

| **First Component** | |
|---|---|
| **Ingredient** | **% w/w** |
| DI Water | 55.398 |
| Calcium Nitrate, USP/ACS | 0.750 |
| Potassium Nitrate, USP/FCC | 5.000 |
| Phosphoric Acid, 75% | 4.502 |
| Pluronic F-127, NF Prill | 20.000 |
| L-Arginine, USP | 12.000 |
| Sodium Saccharin USP/FCC | 0.250 |
| Natural Peppermint Oil, Food | 2.100 |
| First Component Compounding Note: In the blending vessel of a double planetary mixer or similar mixer that fits for low shear processing of viscous materials, add Pluronic F-127, natural peppermint oil, and DI water solution of calcium nitrate, potassium nitrate, phosphoric acid (75%), L-Arginine, sodium saccharin. Then close the blending vessel and start initial mixing at low speed for around 30 minutes. Then stop mixing and let the mixture continue its thickener's hydration in the blending vessel for approximately 1 hour 45 minutes. Then restart mixing under around 1 bar vacuum for another 1.0 hour before a homogenous hydrogel of the first component is produced. | |

| **Second Component** | |
|---|---|
| **Ingredient** | **% w/w** |
| DI Water | 69.498 |
| Potassium Hydroxide USP/NF | 2.860 |
| Disodium Phosphate USP/FCC | 0.375 |
| Monosodium Phosphate USP/FCC | 0.375 |
| Potassium Nitrate USP/FCC | 5.000 |
| Sodium Saccharin USP/FCC | 0.250 |
| Sodium Fluoride USP | 0.442 |
| Pluronic F-127 NF Prill | 20.000 |
| Natural Peppermint Oil, Food | 1.200 |
| Second Component Compounding Note: In the blending vessel of a double planetary mixer or similar mixer that fits for low shear processing of viscous materials, add Pluronic F-127, natural peppermint oil, and DI water solution of potassium hydroxide, disodium phosphate, monosodium phosphate, potassium nitrate, sodium saccharin, and sodium fluoride. Then close the blending vessel and start initial mixing at low speed for around 30 minutes. Then stop mixing and let the mixture continue its thickener's hydration in the blending vessel for approximately 1 hour 45 minutes. Then restart mixing under around 1 bar vacuum for another 1 hour before a homogenous hydrogel of the second component is produced. | |

Cationic ACP Generation: In the mixing tip or mixing chamber of a dual-barrel syringe, dual-chamber bottle, or a dispensing device with two compartments (one barrel/chamber/compartment is filled with the first component hydrogel and another one is filled with the second component hydrogel, respectively) from Table 3, mixing the two parts' hydrogels according to 1: 1 (v/v) mixing ratio to create a final on-site formed cationic arginine ACP hydrogel for dental treatment of hypersensitive teeth.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A composition for treating dentin hypersensitivity, comprising:
a first component comprising: (i) a first rheology modifying polymer; (ii) a calcium ion source; (iii) a positively charged basic amino acid; and (iv) an acidifying agent, wherein the first component has a pH in the range of about 2.0 to about 7.0;
a second component comprising: (i) a second rheology modifying polymer; (ii) a phosphate ion source; and (iii) an alkalizing agent, wherein the second component has a pH in the range of about 7.0 to about 14.0;
wherein the first component and the second component are combined into a mixture such that a cationic amorphous calcium phosphate gel composition is created instantly when the pH of the mixture reaches a pH range of about 7.0 to about 9.5.

2. The composition of claim 1, wherein the first component and the second component are combined into a mixture via a mixing tip or a mixing chamber.

3. The composition of claim 1, wherein the first and second rheology modifying polymer is one or more of a poloxamer, carbomer, hydroxyethylcellulose, xanthan gum, and polyvinylpyrrolidone.

4. The composition of claim 1, wherein the calcium ion source is a calcium salt of nitric acid, a calcium salt of aspartic acid, a calcium salt of glutamic acid, or a water-soluble calcium salt of another suitable acid that is not toxic.

5. The composition of claim 1, wherein the positively charged basic amino acid is one or more of lysine, arginine, and histidine.

6. The composition of claim 1, wherein the acidifying agent is phosphoric acid, or other acid compatible with ingredients of the first component, such that the first component has a pH in the range of about 2.0 to about 7.0.

7. The composition of claim 1, wherein the phosphate ion source is disodium phosphate, monosodium phosphate, or other suitable alkaline water-soluble phosphate salt.

8. The composition of claim 1, wherein the alkalizing agent is sodium hydroxide, potassium hydroxide, or other suitable alkaline compound, such that the second component has a pH in the range of about 7.0 to about 14.0.

9. The composition of claim 1, wherein the first component and/or the second component comprises one or more of a flavoring agent, a sweetener, a solvent, a surfactant, fluoride, and a nerve desensitizing agent.

10. The composition of claim 9, wherein the nerve desensitizing agent is a potassium salt.

11. An oral health care method (100) for treating dentin hypersensitivity, comprising:
providing (120) a first component comprising: (i) a first rheology modifying polymer; (ii) a calcium ion source; (iii) a positively charged basic amino acid; and (iv) an acidifying agent, wherein the first component has a pH in the range of about 2.0 to about 7.0;
providing (130) a second component comprising: (i) a second rheology modifying polymer; (ii) a phosphate ion source; and (iii) an alkalizing agent, wherein the second component has a pH in the range of about 7.0 to about 14.0, wherein the first component and the second component are separately housed prior to mixing;
combining (140) the first component and the second component together by simultaneously dispensing the first component and the second component into a mixing tip or chamber thereby instantly forming a cationic amorphous calcium phosphate (ACP) gel composition when the pH of the mixture reaches a pH range of about 7.0 to about 9.5; and
applying (150) the instantly formed cationic amorphous calcium phosphate gel composition onto the dentin surface of the user's teeth.

12. The oral health care method of claim 11, wherein the pH of the first component is in the range of about 4.0 to about 6.5.

13. The oral health care method of claim 11, wherein the pH of the second component is in the range of about 8.0 to about 11.5.

14. A dosing device (200) for preparing a composition for treatment of dentin hypersensitivity, comprising:
a first compartment (26) containing a first component comprising: (i) a first rheology modifying polymer; (ii) a calcium ion source; (iii) a positively charged basic amino acid; and (iv) an acidifying agent, wherein the first component has a pH in the range of about 2.0 to about 7.0, and
a second compartment (28) containing a second component comprising: (i) a second rheology modifying polymer; (ii) a phosphate ion source; and (iii) an alkalizing agent, wherein the second component has a pH in the range of about 7.0 to about 14.0; and
a dispensing portion (14) configured to receive the first component and the second component into a mixing tip or chamber (20) and to combine the first component and the second component into a mixture creating a cationic amorphous calcium phosphate gel composition instantly when the pH of the mixture reaches a pH range of about 7.0 to about 9.5, and wherein the dispensing device has an opening to apply the cationic amorphous calcium phosphate gel composition onto a dentin surface of a user's teeth.

15. The system of claim 14, wherein the dosing device includes multiple compartments, each compartment comprising one or more compatible ingredients of the first component and/or one or more compatible ingredients the second component, and wherein the content of the multiple compartments is dispensed into the mixing tip or chamber to form the cationic amorphous calcium phosphate gel composition.
